# FASCICULE DE BREVET EUROPEEN

(11) **EP 3 654 011 B1**
(45) Date de publication et mention de la délivrance du brevet: **29.06.2022**
(21) Numéro de dépôt: 19207805.3
(22) Date de dépôt: 07.11.2019
(51) Int. Cl.: G01N 1/40, G01N 33/02, G01N 33/04, G01N 33/12, G01N 1/38

(54) **PROCÉDÉ DE PRÉPARATION D'UN ÉCHANTILLON À ANALYSER OBTENU À PARTIR DE MATRICE ALIMENTAIRE**
PRÄPARATIONSVERFAHREN EINER ZU ANALYSIERENDEN PROBE, DIE AUS EINER LEBENSMITTELMATRIX ERHALTEN WURDE
METHOD FOR PREPARING A SAMPLE TO BE ANALYSED OBTAINED FROM A FOOD MATRIX

(30) Priorité: 16.11.2018 FR 1860598
(43) Date de publication de la demande: 20.05.2020
(73) Titulaire: Commissariat à l'Énergie Atomique et aux Énergies Alternatives, 75015 Paris (FR)
(72) Inventeur: BAQUE, Mélissa, 38054 Grenoble cedex 09 (FR); BOURDAT, Anne-Gaëlle, 38054 Grenoble cedex 9 (FR)
(74) Mandataire: INNOV-GROUP

(56) Documents cités:
- US-A1- 2018 180 611
- US-B2- 7 625 707
- US-B2- 8 927 290
- X. LI ET AL: "Rapid Sample Processing for Detection of Food-Borne Pathogens via Cross-Flow Microfiltration", APPLIED AND ENVIRONMENTAL MICROBIOLOGY, vol. 79, no. 22, 6 septembre 2013 (2013-09-06), pages 7048-7054, XP055340896, US ISSN: 0099-2240, DOI: 10.1128/AEM.02587-13

## Description

### Domaine technique de l'invention

L'invention concerne un procédé de préparation d'un échantillon en vue de réaliser une analyse d'espèces biologiques ciblées, telles que par exemple des bactéries dans une matrice alimentaire ou dans la chaîne de fabrication d'une matrice alimentaire. Par matrice alimentaire, on entend de manière non limitative un échantillon de produits tels que du saumon, du lait (notamment infantile en poudre), du steak haché, du jambon...

### Etat de la technique

La détection des bactéries dans un échantillon alimentaire sur la chaîne de fabrication est devenue aujourd'hui nécessaire pour des questions d'hygiène et de respect des normes et de réglementation.

Les méthodes employées actuellement sont souvent longues à mettre en œuvre et sont donc très souvent peu efficaces et d'un rendement insuffisant car elles nécessitent un volume de matrice alimentaire particulièrement conséquent pour obtenir un échantillon final qui comporte suffisamment de bactéries.

Le faible niveau de contamination des cellules cibles, associé à la complexité et à la diversité des matrices alimentaire, est un réel challenge pour le développement d'une technique de détection. En effet, les aliments sont des échantillons complexes, comportant non seulement une multitude de microorganismes, mais aussi des protéines, des graisses ou des débris pouvant interférer avec la méthode de détection. Les techniques de détection sont pour la plupart d'entre elles, rapides et sensibles mais nécessitent un temps d'enrichissement important (entre 7h et 26h) pour atteindre un nombre de cellules cibles suffisant. Les techniques de détection sont aussi limitées par le volume de prise d'essai, souvent trop faible, ce qui conduit à une mauvaise représentativité de l'ensemble de l'échantillon lorsque celui-ci est peu contaminé.

Il en ressort qu'il existe un besoin d'établir un procédé de préparation adapté à l'analyse qualitative et quantitative des bactéries présentes dans un prélèvement de alimentaire ou dans un prélèvement réalisé sur la chaîne de fabrication d'un produit alimentaire, ce procédé de préparation permettant d'obtenir in fine un échantillon à analyser de manière rapide et avec un rendement très satisfaisant, c'est-à-dire avec une concentration élevée de bactéries à analyser à partir d'un prélèvement initial en quantité limitée.

La demande de brevet US2018/180611A1 décrit une méthode de détection de pathogènes dans des échantillons alimentaires. Cette méthode utilise notamment des membranes fibreuses pour effectuer la séparation des espèces biologiques.

### Exposé de l'invention

Ce but est atteint par un procédé de préparation d'un échantillon à partir d'un prélèvement de matrice alimentaire ou d'un prélèvement réalisé sur une chaîne de fabrication de produit alimentaire, selon la revendication 1. D'autres modes de réalisation sont décrits dans les revendications dépendantes.

L'invention concerne également l'utilisation du procédé selon la revendication 1, pour préparer un échantillon contenant des espèces biologiques à analyser selon une technique d'analyse choisie parmi q-PCR, LAMP, séquençage, détection immunologique.

Pour les raisons de complexité évoqués ci-dessus, il faut noter que la filtration est une technique intéressante pour traiter un grand volume d'échantillon ainsi que pour concentrer et purifier les pathogènes cibles.

### Brève description des figures

D'autres caractéristiques et avantages vont apparaître dans la description détaillée qui suit faite en regard des dessins annexés dans lesquels :
- La figure 1 représente un diagramme montrant les différentes étapes du procédé de préparation de l'invention.
- La figure 2 illustre de manière schématique les différentes étapes de filtrage mises en œuvre dans le procédé de préparation de l'invention.
- La figure 3A représente un dispositif de préparation pouvant être employé dans la mise en œuvre du procédé.
- La figure 3B représente la mise en série de deux dispositifs tels que celui de la figure 3A.
- La figure 3C illustre le principe d'interchangeabilité des filtres dans le dispositif de la figure 3A.
- La figure 4 illustre, par deux diagrammes, l'intérêt d'ajout d'une anti-protéase dans le procédé de l'invention.

### Description détaillée d'au moins un mode de réalisation

L'invention concerne un procédé de préparation d'un échantillon en vue de la détection d'espèces biologiques présentes dans un prélèvement de matrice alimentaire ou dans un prélèvement réalisé sur une chaîne de fabrication de produit alimentaire. Dans ce dernier cas, il ne s'agira donc pas forcément d'un prélèvement de matrice alimentaire mais d'un prélèvement d'un film ou équivalent sur la chaîne de fabrication. De manière non limitative, on considérera que le procédé est essentiellement adapté pour un prélèvement direct de la matrice alimentaire.

La matrice alimentaire servant de base au prélèvement à analyser peut être prélevée directement sur la chaîne de fabrication ou dans le produit fini.

De manière non limitative, la matrice alimentaire peut être constituée de lait (notamment infantile), jambon, steak haché, saumon, épinards ou même de croquettes pour animaux.

Les espèces biologiques ciblées sont essentiellement des bactéries mais il pourrait également s'agir de tous types de microorganismes ou de molécules d'ADN. Dans la suite de la description, le procédé sera décrit plus précisément pour des espèces biologiques de type bactéries.

De manière non limitative, l'analyse postérieure des espèces biologiques pourra être mise en œuvre selon différentes techniques connues telles que Q-PCR (Polymerase Chain Reaction), LAMP, séquençage...

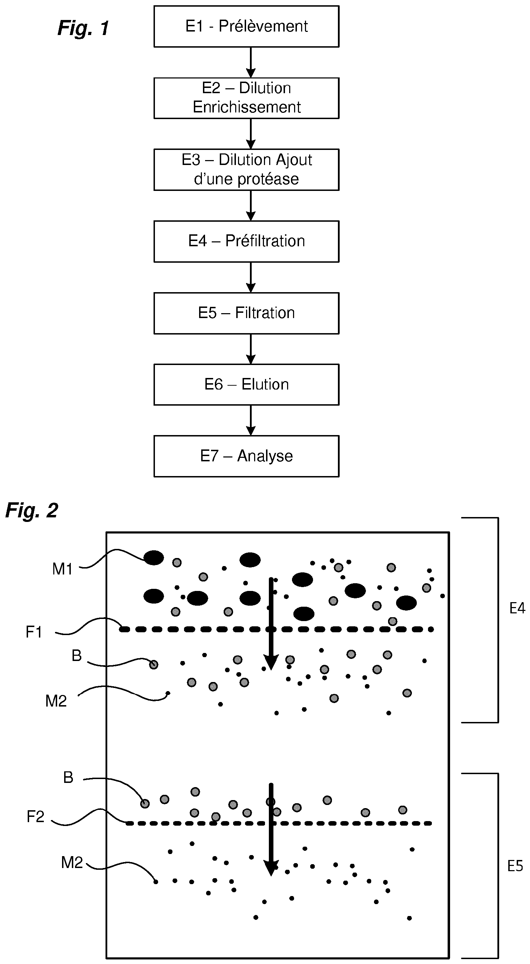

En référence aux figures 1 et 2, selon l'invention, le procédé de préparation d'un échantillon pour détection de bactéries comporte les étapes suivantes décrites ci-dessous.

### Etape E1

Il s'agit d'une étape de prélèvement de la matrice alimentaire à analyser. Comme évoqué ci-dessus, celle-ci pourra être de tous types.

### Etape E2

En règle générale, on pratique une première dilution de la matrice alimentaire prélevée dans un liquide d'enrichissement afin d'obtenir une prolifération des bactéries (par exemple 25g du produit alimentaire qsp 250mL de liquide du milieu d'enrichissement)

### Etape E3

Il s'agit d'une étape clé du procédé de préparation de l'invention qui consiste à réaliser une deuxième dilution de l'échantillon déjà dilué lors de l'étape précédente avec un liquide contenant une protéase.

Les matrices alimentaires sont en effet des échantillons complexes, comportant non seulement une multitude de microorganismes naturellement présents, mais aussi des débris, des graisses ou encore des protéines qui limitent la filtration d'un grand volume de l'échantillon, car les filtres sont rapidement colmatés. Le principe est donc d'utiliser la protéase pour dégrader les petits éléments présents dans la matrice ou à la surface des bactéries pour les dissocier (bactéries-particules, particules-particules) et permettre une meilleure filtration de l'échantillon. De manière avantageuse, la protéase est ainsi amenée à dégrader les protéines et les petits éléments protéiques sans affecter la viabilité des bactéries.

De manière non limitative, la protéase peut être choisie parmi les endopeptidases ou exopeptidases, comme la trypsine, chymotrypsine, la thrombine et la papaïne, l'endolyse C...

Dans la suite de la description, de manière non limitative, la protéase qui est employée est la trypsine.

### Etape E4

Il s'agit d'une étape de préfiltration de l'échantillon obtenu après dilution avec la protéase.

Cette étape de préfiltration est mise en œuvre en employant un premier filtre F1 présentant des pores ayant une taille suffisante pour séparer les molécules M1 de la matrice alimentaire qui sont de grosses tailles par rapport au reste de l'échantillon.

### Etape E5

Il s'agit de l'étape principale de filtration de l'échantillon qui est obtenu après l'étape précédente de préfiltration. Cette étape de filtration est mise en œuvre en employant un deuxième filtre F2 présentant des pores d'une taille inférieure à celle des pores du premier filtre F1 employé lors de la préfiltration.

Ainsi, lors de la filtration, les molécules M2 de matrice alimentaire les plus petites passent au travers du filtre et les espèces biologiques ciblées, c'est-à-dire les bactéries B, restent au-dessus de ce filtre.

### Etape E6 (optionnelle)

Les bactéries B présentes au-dessus du filtre sont éluées afin d'être recueillies pour analyse.

La protéase ajoutée initialement permet également d'améliorer l'élution des espèces biologiques telles que les bactéries concentrées sur le filtre F1. L'ajout de la protéase permet en effet de limiter l'adsorption non spécifique des bactéries sur les surfaces, notamment celles du filtre et donc d'assurer une récupération maximale de bactéries.

L'échantillon final obtenu est donc ainsi prêt pour l'analyse.

### Etape E7

Il s'agit de l'étape d'analyse de l'échantillon obtenu précédemment. L'analyse pourra être réalisée selon différentes techniques évoquées ci-dessus.

De manière non limitative, le procédé de préparation pourra être mis en œuvre en respectant un ou plusieurs des principes suivants :
- Le premier filtre F1 peut être doté de pores ayant une taille comprise entre 1µm et 8µm, avantageusement comprise entre 3µm et 6µm, avantageusement de 5µm.
- Le deuxième filtre F2 peut être doté de pores ayant une taille comprise entre 0.2µm et 1µm, avantageusement comprise entre 0.4µm et 0.8µm, avantageusement de 0.6µm ou de 0.8µm selon la technique d'analyse employée postérieurement.
- Le procédé peut être mis en œuvre, au moins à partir de l'étape de filtration, en utilisant un dispositif de préparation tel que décrit dans la demande de brevet EP3222989A1**.**

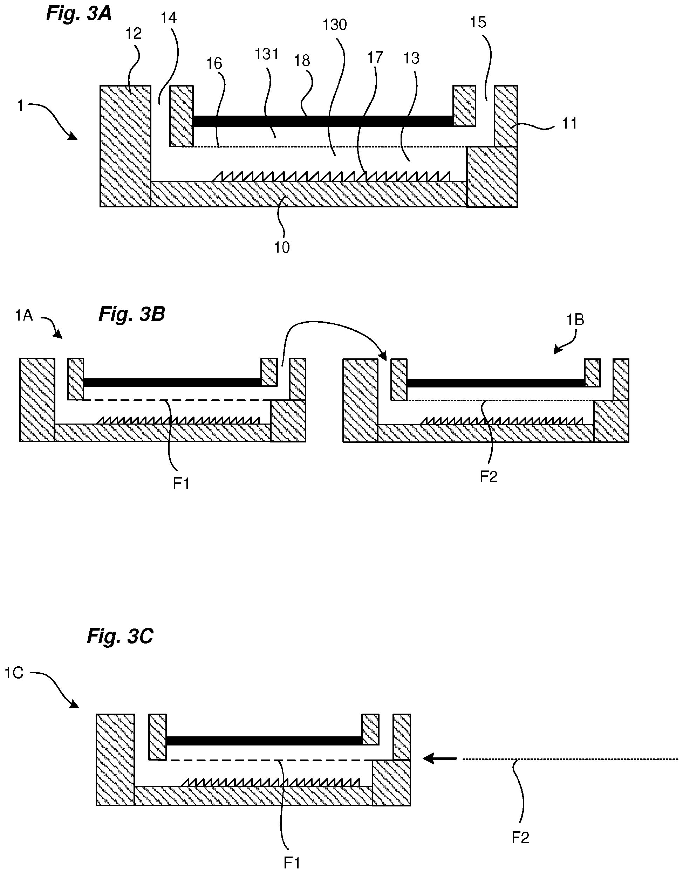

En référence à la figure 3A, ce dispositif de préparation 1 comporte un boîtier comprenant une paroi inférieure 10, une paroi latérale 11 et une paroi supérieure 12. Toutes les parois du boîtier seront préférentiellement réalisées dans un même matériau. Ce matériau sera notamment apte à pouvoir subir un chauffage dans une fourchette de température comprise entre 20°C et 100°C. Préférentiellement, certaines parois du boîtier, au moins sa paroi latérale, seront réalisées dans un matériau transparent. Préférentiellement, le matériau employé sera un plastique, par exemple de type PMMA (Poly(Méthacrylate de Méthyle)).

Le dispositif 1 comporte une chambre 13 ménagée dans le boîtier. Cette chambre représente l'emplacement dans lequel peuvent être effectuées à la fois la purification/concentration, la lyse mécanique, la séparation et éventuellement la détection dans les espèces biologiques. La chambre 13 est fermée vers le bas par la paroi inférieure du boîtier.

Le dispositif comporte un premier canal 14 pour injecter des fluides dans la chambre ou pour évacuer des fluides en dehors de la chambre. Le premier canal 14 comporte une première extrémité comportant une ouverture ménagée par exemple à travers la paroi supérieure 12 du boîtier et une deuxième extrémité qui débouche dans ladite chambre 13. La première extrémité du premier canal 14 est par exemple agencée verticalement et sa deuxième extrémité débouche par exemple horizontalement dans la chambre 13. La première extrémité du premier canal est par exemple évasée pour y appliquer le cône d'une pipette ou sera adaptée au type de dispositif employé pour injecter le fluide dans le dispositif. A titre d'exemple, il peut s'agir d'une ouverture présentant un embout de type lueur pour y connecter une seringue ou adaptée pour y connecter un circuit fluidique tel que celui qui sera décrit ci-après.

Le dispositif comporte un deuxième canal 15. Ce deuxième canal 15 comporte également une première extrémité qui communique avec l'extérieur, formant une ouverture réalisée par exemple à travers la paroi supérieure du boîtier et une deuxième extrémité qui communique avec l'espace formé par la chambre 13. Par ce deuxième canal 15, on peut également injecter des fluides dans ladite chambre ou évacuer des fluides en dehors de ladite chambre. Sa première extrémité est par exemple agencée verticalement et sa deuxième extrémité horizontalement. La chambre 13 est placée entre le premier canal 14 et le deuxième canal 15. De manière identique, la première extrémité de ce deuxième canal est par exemple évasée pour y appliquer le cône d'une pipette ou sera adaptée au type de dispositif employé pour injecter le fluide dans le dispositif. A titre d'exemple, il peut s'agir d'une ouverture présentant un embout de type lueur pour y connecter une seringue ou adaptée pour y connecter un circuit fluidique.

Vers le haut, la chambre 13 peut être fermée par une membrane 18 souple et étirable, préférentiellement transparente. La paroi supérieure 12 du boîtier du dispositif comporte ainsi une ouverture qui est recouverte de manière hermétique par ladite membrane 18. Ladite membrane est ainsi ancrée dans le boîtier par toute solution de fixation adaptée, par exemple par collage. Cette membrane 18 sera par exemple composée d'un film, par exemple de type MicroAmp, 3M (marques déposées), d'épaisseur, de dimensions et de constitution adaptées pour se déformer de manière élastique, par rapport à ses points d'ancrage, notamment jusqu'au fond de la chambre 13.

Par le terme "transparent", on entend que le matériau employé est au moins partiellement transparent à la lumière visible, de manière à laisser passer au moins 80% de cette lumière. Il faut ainsi comprendre qu'il sera suffisamment transparent pour voir l'intérieur de la chambre 13, au moins le deuxième espace situé au-dessus du filtre.

Le dispositif comporte un filtre 16 agencé dans ladite chambre 13 et séparant ladite chambre 13 en deux espaces. Les deux espaces sont par exemple superposés et désignés ainsi espace inférieur 130 situé sous le filtre et espace supérieur 131 situé au-dessus du filtre. Ce filtre 16 est préférentiellement réalisé en tout ou partie sous la forme d'un film souple et fin, tiré dans l'espace formé par la chambre de manière à ne permettre le passage d'un espace à l'autre que par les pores du filtre 16. Le film présente une déformabilité élastique lui permettant de s'étirer lors de l'exercice d'une force d'appui dans une direction sensiblement verticale, cette déformabilité élastique ayant un niveau suffisant pour atteindre la surface inférieure de la chambre 13. Le filtre 16 présente un diamètre moyen de pores tel que défini ci-dessus. Le diamètre des pores est bien entendu adapté pour assurer une séparation entre différentes espèces biologiques présentes dans l'échantillon. Le filtre 16 sera par exemple composé d'un film d'épaisseur, de dimensions et de constitution adaptée pour se déformer jusqu'au fond de la chambre 13 par rapport à ses points d'ancrage. Selon un mode de réalisation particulier, le filtre pourra être aussi réalisé dans un matériau transparent, par exemple avec les mêmes caractéristiques de transparence que la membrane.

Le dispositif peut avantageusement comporter une surface d'appui rugueuse 17 agencée sur le fond de la chambre 13. Cette surface d'appui rugueuse 17 s'étend sur une partie majoritaire du fond de la chambre. Elle comporte un paramètre de rugosité de surface moyen compris entre 0.1µm et 10 µm, préférentiellement compris entre 0.2 µm et 3 µm. Cette surface d'appui rugueuse 17 est destinée à permettre une lyse mécanique des espèces biologiques présentes dans un échantillon biologique placé dans le dispositif. Préférentiellement, la lyse mécanique est réalisée en broyant lesdites espèces biologiques, par abrasion sur ladite surface d'appui rugueuse. L'opération de broyage est mise en œuvre par un mouvement de friction des espèces biologiques contre la surface d'appui rugueuse, en employant un organe de broyage adapté. Cet organe sera par exemple une spatule ou une tige, par exemple en matériau plastique ou métallique. Cet organe est appliqué de l'extérieur de la chambre 13 et son extrémité est appliquée contre la surface externe de la membrane 18 de manière à étirer la membrane 18 et le filtre vers le fond de la chambre et ainsi frictionner les espèces biologiques présentes dans un échantillon contre la surface d'appui rugueuse.

Préférentiellement, le boîtier peut avantageusement intégrer des moyens de chauffage de l'espace interne de la chambre, composés par exemple d'au moins une résistance 19 chauffante ou d'un élément Peltier, comme représenté sur les figures annexées. La résistance est par exemple fixée sous la paroi inférieure du boîtier. Une source d'alimentation 20 sera par exemple prévue pour alimenter la résistance 19. La source d'alimentation comportera par exemple une ou plusieurs piles électriques, fournissant suffisamment d'énergie pour chauffer la chambre à une température comprise dans la fourchette définie ci-dessus, c'est-à-dire de 20°C à 100°C. Bien entendu, d'autres moyens de chauffage pourraient être employés, comprenant par exemple une encre conductrice déposée par imprimerie ou sérigraphie sous la paroi inférieure du boîtier.

Ainsi, pour résumer, le dispositif peut avantageusement comporter la structure "multicouches" suivante :
- Une surface inférieure d'appui rugueuse 17,
- Un espace inférieur 130 d'une chambre 13 situé au-dessus de la surface d'appui rugueuse 17,
- Un filtre 16 souple et étirable situé au-dessus de l'espace inférieur 130,
- Un espace supérieur 131 de la chambre 13 situé au-dessus du filtre 16,
- Une membrane 18 souple et étirable située au-dessus de l'espace supérieur 131, fermant hermétiquement la chambre et accessible depuis l'extérieur du dispositif.

Lors de la filtration réalisée à l'étape E5 décrite ci-dessus, l'échantillon est injecté sous forme liquide à travers le canal 15 du dispositif afin de traverser le filtre 16 dans un sens. Une fois la filtration terminée, les bactéries B sont retenues par le filtre 16 et restent dans l'espace supérieur 131 de la chambre 13, tandis que les molécules M2 passent à travers le filtre 16 (qui correspond alors au filtre F2 décrit ci-dessus) et migrent vers l'espace inférieur 130 de la chambre 13. L'étape E6 d'élution des bactéries B est ensuite mise en œuvre en injectant un liquide d'élution par le canal 15 du dispositif de manière à passer à travers le filtre dans le sens opposé et à emporter les bactéries B uniquement vers l'extérieur par le canal 14 du dispositif. On peut noter que si l'objectif est d'éluer les molécules d'ADN obtenues après une lyse des espèces biologiques contenues dans l'échantillon, le liquide d'élution serait alors introduit en sens inverse, c'est-à-dire du canal 14 vers le canal 15.

L'étape E6 d'élution est cependant optionnelle car le dispositif permet, grâce à son architecture transparente, de mener une analyse de type q-PCR in situ, c'est-à-dire directement dans le dispositif.

Comme représenté sur la figure 3B, l'étape de préfiltration peut également être mise en œuvre en employant un dispositif 1a de préparation du même type connecté en série et en amont, doté du filtre F1 et d'un dispositif 1b similaire doté du filtre F2 employé pour la filtration et l'élution. Les deux dispositifs pourront être réalisés sur une même carte et communiquer entre eux via un circuit fluidique. En variante représentée sur la figure 3C, il serait également possible d'employer un seul dispositif 1c tel que celui décrit ci-dessus dont le filtre est amovible. Cette solution permettrait de conserver l'échantillon dans le même dispositif. Le premier filtre F1 serait d'abord installé dans le dispositif 1c pour l'étape de préfiltration puis retiré et remplacé par le deuxième filtre F2 pour effectuer l'étape de filtration.

Grâce aux parties transparentes, le dispositif de préparation peut être directement employé pour une analyse de type q-PCR, un test immunologique, ou un séquençage.

En cas d'analyse ultérieure de type q-PCR, il peut s'avérer nécessaire de stopper l'action de la protéase. Selon l'invention, il est nécessaire d'inhiber l'action de la protéase avant l'étape de pré-filtration.

L'action de la protéase peut être stoppée par des procédés de type thermique, une acidité ou des inhibiteurs de protéases chimiques ou biochimiques, tels que par exemple :
- Pepstatine, leupeptine, sérum de veau fœtal (qui peut contenir de l'alpha-antitrypsine, ou des cocktails d'enzymes comme le « complete » de Roche (marque déposée)) ;
- Le fluorure de phénylméthylsulfonyle (PMSF) ;

De manière non limitative, l'expérimentation peut se dérouler de la manière suivante :
- Dilution de la matrice alimentaire après enrichissement au ½ dans de la trypsine 0,05%-0,125%.
- En prévision d'une analyse q-PCR, il est possible de stopper la réaction avec un inhibiteur de protéase (SVF) qui est ajouté au 1/10 du volume de la trypsine dans l'échantillon.
- Préfiltration : Un grand volume d'échantillon (1-10mL) est ensuite pré filtré sur un filtre commercial (5µm, 1,2µm ou 2µm) laissant passer les plus petites molécules (M2+B) et retenir les plus grosses molécules (M1).
- Une filtration plus fine permettant de retenir et concentrer les bactéries B est ensuite réalisée sur un dispositif de préparation tel que celui décrit ci-dessus et portant un filtre ayant des pores de taille 0,6µm ou 0,8µm.

Divers essais du procédé de l'invention ont été mis en œuvre, sur des matrices alimentaires différentes, permettant de valider notamment le principe d'ajout de la protéase en vue d'une meilleure filtration.

### 1. Matrice Alimentaire de type SAUMON

La préfiltration du saumon tel quel ou dilué dans un tampon salin (PBS) au même ratio que la trypsine ne permet pas de passer un grand volume à travers le filtre, 2mL sur un filtre de 5µm de taille de pores lors de l'étape de préfiltration, puis 50µL sur un filtre de 0.6µm de taille de pores lors de l'étape principale de filtration. Lorsque la trypsine est ajoutée, la filtration d'un grand volume devient alors possible, puisque un volume de 10mL de l'échantillon a été passé sur le premier filtre (5µm) lors de l'étape de préfiltration et de 2mL sur le deuxième filtre (0.6µm) lors de l'étape principale de filtration. On peut ainsi noter que lorsque la matrice est diluée dans une solution de trypsine, la filtration est plus efficace. Les résultats obtenus sont repris dans le tableau suivant :

| **Matrice** | **Dilution de la matrice** | **Diluant** | **Filtres (Volume éluat/Volume injecté)** | | |
|---|---|---|---|---|---|
| Saumon | 0 | **-** | 5µm | 2µm | 1.2µm |
| | | | 2ml/5ml | 0.5ml/5ml | 0.850ml |
| Saumon | ½ | PBS | Préfiltration (5µm) 2ml Deuxième filtration (0.6µm) 50µl | | 1.2µm 0.990ml |
| Saumon | ½ | Trypsine (0.05%) | Préfiltration (5µm) 10ml/10ml Deuxième filtration (0.6µm) 2ml/2ml | | Préfiltration (1.2µm) 10ml/10ml Deuxième filtration (0.6µm) 2ml/2ml |
| Saumon | 1/10 | Trypsine (0.05%)/EDTA | Préfiltration (5µm) 10ml/10ml Deuxième filtration (0.6µm) 10ml/10ml | | |
| Saumon | 0 | Trypsine (0.05%)/EDTA | 5µm 5ml/5ml | 2µm 5ml/5ml | |

Une observation au microscope des solutions révèle qu'après action de la trypsine, le nombre de petites particules diminue fortement. Après passage sur le premier filtre lors de la préfiltration, les grosses particules sont retenues. Il ne resteque des toutes petites particules qui sont retrouvées après passage sur le deuxième filtre (0.6µm), indiquant bien que ces petites particules ne vont pas colmater le filtre (ou en tous les cas nettement moins).

### 2. Matrice alimentaire de type bœuf après enrichissement

Pour cette matrice alimentaire, on obtient le tableau suivant :

| Matrice | **Dilution de la matrice** | Diluant | Filtres (Volume éluat/Volume injecté) | | | |
|---|---|---|---|---|---|---|
| Bœuf | 0 | - | Préfiltration (2 µm) 1.5ml/5ml Deuxième filtration (0.6µm) 0.2ml | 1.2µm 2ml/5 ml | 2µm 1.5ml/5 ml | Préfiltration (5 µm) 5ml/5ml Deuxième filtration (0.6µm) 0.7ml |
| Bœuf | 1/2 | PBS | Préfiltration (2 µm) 5ml/5ml Deuxième filtration (0.6µm) 0.4ml | | | |
| Bœuf | 1/10 | Trypsine (0.05%)/ED TA | | | 1.2µm 20ml/20 ml | Préfiltration (5 µm) 10ml/10ml Deuxième filtration (06µm) 10ml/10ml |
| Bœuf | 1/2 | Trypsine (0.05%) | Préfiltration (2µm) 10ml/10ml Deuxième filtration (0.6µm) 1.5ml/2ml | | | Préfiltration (5 µm) 10ml/10ml Deuxième filtration (0.6µm) 2ml/2ml |

Des essais similaires ont été réalisés sur du lait infantile (en poudre) et des épinards, pour des résultats et conclusions similaires.

Par ailleurs, en plus de faciliter la filtration de la matrice alimentaire, on a constaté que l'ajout de la trypsine permet également d'augmenter le nombre de bactéries obtenues après l'étape d'élution E6 et donc d'améliorer le rendement. Le tableau ci-dessous permet de mettre en évidence cet avantage :

| | **Avant injection** | **Elution** | **Rendement** |
|---|---|---|---|
| **Bactéries sans trypsine** | 8,80E+05 | 1,90E+05 | 21,59% |
| | 8,80E+05 | 2,40E+05 | 27,27% |
| | 8,50E+05 | 3,10E+05 | 36,47% |
| | 8,50E+05 | 1,05E+05 | 12,35% |
| **Bactéries avec trypsine** | 4,25E+05 | 2,90E+05 | 68,24% |
| | 4,25E+05 | 3,55E+05 | 83,53% |
| | 6,50E+05 | 5,15E+05 | 79,23% |
| | 6,50E+05 | 3,85E+05 | 59,23% |

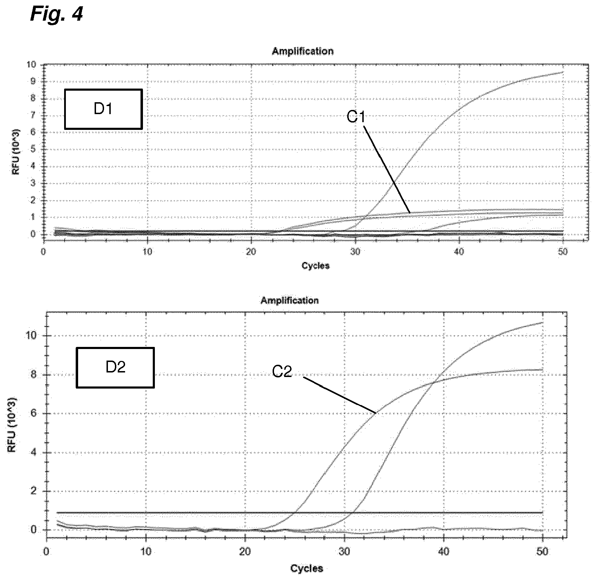

La figure 4 met en évidence l'intérêt de l'ajout d'une anti-protéase de type chimique lorsqu'une analyse de type Q-PCR doit être menée par la suite.

Sur cette figure, on peut voir que, lorsqu'on réalise une analyse Q-PCR après filtration-concentration-purification et lyse des bactéries, le signal de fluorescence est aplati (diagramme D1 et courbe C1). Pour pallier l'effet de la trypsine et rétablir le signal, l'anti-protéase est ajoutée avant l'analyse. Sur le diagramme D2, le signal formé par la courbe C2 est rétabli.

La solution de l'invention présente ainsi de nombreux avantages, parmi lesquels :
- Elle permet d'éviter un colmatage trop rapide du filtre et donc d'améliorer la filtration et de passer un volume plus important de liquide à travers le filtre ;
- Elle est compatible avec toutes les techniques d'analyse connues ;
- Elle n'affecte pas les bactéries et ne les dégrade pas ;
- Elle ne présente pas de difficultés particulières de mise en oeuvre ;
- Elle permet un gain de temps, notamment grâce à une filtration améliorée ;

## Revendications

1. Procédé de préparation d'un échantillon à partir d'un prélèvement de matrice alimentaire ou d'un prélèvement réalisé sur une chaîne de fabrication de produit alimentaire, ledit prélèvement contenant des espèces biologiques à analyser, comportant
- Une première étape (E3) de dilution d'un premier échantillon issu dudit prélèvement avec une protéase pendant une durée suffisante pour dégrader ledit premier échantillon et obtenir un deuxième échantillon liquide,
- Une deuxième étape (E4) de préfiltration dudit deuxième échantillon liquide à travers un premier filtre (F1) pour séparer des premières molécules (M1) qui sont de tailles supérieures à celle des portes dudit premier filtre (F1), en vue d'obtenir un troisième échantillon comprenant les espèces biologiques à analyser et des deuxièmes molécules (M2) de tailles inférieures à celles des pores dudit premier filtre,
- Une troisième étape (E5) de filtration dudit troisième échantillon à travers un deuxième filtre (F2) pour séparer lesdites espèces biologiques desdites deuxièmes molécules (M2),
**caractérisé en ce qu'**il comporte une étape d'inhibition de la protéase, mise en œuvre entre la première étape (E3) de dilution et la deuxième étape (E4) de préfiltration.

2. Procédé selon la revendication 1, **caractérisé en ce qu'**il comporte une étape (E2) préalable de dilution dudit prélèvement dans un milieu d'enrichissement.

3. Procédé selon la revendication 1 ou 2, **caractérisé en ce qu'**il comporte une étape (E6) d'élution des espèces biologiques obtenues après ladite troisième étape de filtration.

4. Procédé selon l'une des revendications 1 à 3, **caractérisé en ce que** la protéase peut être choisie parmi les endopeptidases ou exopeptidases, comme la trypsine, chymotrypsine, la thrombine et la papaïne, l'endolyse C.

5. Procédé selon la revendication 1, **caractérisé en ce que** l'étape d'inhibition de la protéase est mise en œuvre par ajout d'un composé chimique de type anti-protéase.

6. Procédé selon la revendication 5, **caractérisé en ce que** l'anti-protéase peut être choisie parmi la pepstatine, la leupeptine, le sérum de veau foetal, le fluorure de phénylméthylsulfonyle.

7. Procédé selon l'une des revendications 1 à 6, **caractérisé en ce que** le premier filtre (F1) est doté de pores ayant une taille comprise entre 1µm et 6µm.

8. Procédé selon l'une des revendications 1 à 7, **caractérisé en ce que** le deuxième filtre (F2) est doté de pores ayant une taille comprise entre 0.4µm et 0.8µm.

9. Procédé selon l'une des revendications 1 à 8, **caractérisé en ce que** la troisième étape est mise en œuvre employant un dispositif de préparation qui comporte au moins :
- Un boîtier comportant au moins une ouverture,
- Un premier canal (14) ménagé dans ledit boîtier,
- Un deuxième canal (15) ménagé dans ledit boîtier,
- Une chambre (13) dans laquelle débouche le premier canal et le deuxième canal,
- Un filtre (16) séparant ladite chambre en deux espaces distincts de manière à définir un premier espace (130) dans lequel débouche ledit premier canal d'injection et un deuxième espace (131) dans lequel débouche ledit deuxième canal, ledit filtre ayant une porosité adaptée pour retenir lesdites espèces biologiques à analyser.

10. Utilisation du procédé tel que défini dans l'une des revendications 1 à 9, pour préparer un échantillon contenant des espèces biologiques à analyser selon une technique d'analyse choisie parmi q-PCR, LAMP, séquençage, détection immunologique.

## Patentansprüche

1. Verfahren zur Herstellung einer Probe ausgehend von einer Stichprobe einer Lebensmittelmatrix oder einer Stichprobe, die an einer Kette zur Herstellung eines Nahrungsmittelprodukts entnommen wird, wobei die Stichprobe zu analysierende biologische Spezies enthält, das Folgendes umfasst:
- einen ersten Schritt (E3) des Verdünnens einer ersten Probe aus der Stichprobe mit einer Protease für einen Zeitraum, der zum Abbau der ersten Probe und zum Erhalt einer zweiten flüssigen Probe ausreicht,
- einen zweiten Schritt (E4) der Vorfiltration der zweiten flüssigen Probe durch ein erstes Filter (F1), um erste Moleküle (M1) abzutrennen, die größer als die Poren des ersten Filters (F1) sind, um eine dritte Probe zu erhalten, welche die zu analysierenden biologischen Spezies und zweite Moleküle (M2), die kleiner als die Poren des ersten Filters sind, umfasst,
- einen dritten Schritt (E5) der Filtration der dritten Probe durch ein zweites Filter (F2), um die biologischen Spezies von den zweiten Molekülen (M2) abzutrennen,
**dadurch gekennzeichnet, dass** es einen Schritt der Hemmung der Protease umfasst, der zwischen dem ersten Schritt (E3) des Verdünnens und dem zweiten Schritt (E4) der Vorfiltration durchgeführt wird.

2. Verfahren nach Anspruch 1, **dadurch gekennzeichnet, dass** es einen vorgeschalteten Schritt (E2) der Verdünnung der Stichprobe in einem Anreicherungsmedium umfasst.

3. Verfahren nach Anspruch 1 oder 2, **dadurch gekennzeichnet, dass** es einen Schritt (E6) der Elution von nach dem dritten Schritt der Filtration erhaltenen biologischen Spezies umfasst.

4. Verfahren nach einem der Ansprüche 1 bis 3, **dadurch gekennzeichnet, dass** die Protease aus Endopeptidasen oder Exopeptidasen, wie Trypsin, Chymotrypsin, Thrombin und Papain, Endolyse C, ausgewählt sein kann.

5. Verfahren nach Anspruch 1, **dadurch gekennzeichnet, dass** der Schritt der Hemmung der Protease durch die Zugabe einer chemischen Verbindung vom Antiproteasetyp durchgeführt wird.

6. Verfahren nach Anspruch 5, **dadurch gekennzeichnet, dass** die Antiprotease aus Pepstatin, Leupeptin, fetalem Kälberserum, Phenylmethylsulfonylfluorid ausgewählt sein kann.

7. Verfahren nach einem der Ansprüche 1 bis 6, **dadurch gekennzeichnet, dass** das erste Filter (F1) Poren mit einer Größe zwischen 1 µm und 6 µm aufweist.

8. Verfahren nach einem der Ansprüche 1 bis 7, **dadurch gekennzeichnet, dass** das zweite Filter (F2) Poren mit einer Größe zwischen 0,4 µm und 0,8 µm aufweist.

9. Verfahren nach einem der Ansprüche 1 bis 8, **dadurch gekennzeichnet, dass** der dritte Schritt durchgeführt wird, indem eine Herstellungsvorrichtung verwendet wird, die zumindest Folgendes umfasst:
- ein Gehäuse mit mindestens einer Öffnung,
- einen in dem Gehäuse hergestellten ersten Kanal (14),
- einen in dem Gehäuse hergestellten zweiten Kanal (15),
- eine Kammer (13), in die der erste Kanal und der zweite Kanal münden,
- ein Filter (16), das die Kammer derart in zwei getrennte Räume unterteilt, dass ein erster Raum (130), in den der erste Injektionskanal mündet, und ein zweiter Raum (131), in den der zweite Kanal mündet, gebildet werden, wobei das Filter eine Porosität aufweist, die dafür ausgelegt ist, die zu analysierenden biologischen Spezies zurückzuhalten.

10. Verwendung des Verfahrens gemäß der Definition in einem der Ansprüche 1 bis 9 zur Herstellung einer Probe, die biologische Spezies enthält, die gemäß einer aus q-PCR, LAMP, Sequenzierung, immunologischem Nachweis ausgewählten Analysetechnik zu analysieren sind.

## Claims

1. Method for preparing a specimen from a sample of food matrix or a sample taken on a food product production line, said sample containing biological species to be analysed, comprising:
- a first step (E3) of diluting a first specimen of said sample with a protease for a time sufficient to degrade said first specimen and obtain a liquid second specimen,
- a second step (E4) of prefiltering said liquid second specimen through a first filter (F1) in order to separate first molecules (M1) which have sizes greater than those of the pores of said first filter (F1), with a view to obtaining a third specimen comprising the biological species to be analysed and second molecules (M2) having sizes smaller than those of the pores of said first filter,
- a third step (E5) of filtering said third specimen through a second filter (F2) in order to separate said biological species from said second molecules (M2),
**characterized in that** it comprises a protease inhibition step, carried out between the dilution first step (E3) and the prefiltration second step (E4).

2. Method according to Claim 1, **characterized in that** it comprises a prior step (E2) of diluting said sample in an enrichment medium.

3. Method according to Claim 1 or 2, characterising that it comprises a step (E6) of eluting the biological species obtained after said filtration third step.

4. Method according to one of Claims 1 to 3, **characterized in that** the protease may be chosen from endopeptidases or exopeptidases, such as trypsin, chymotrypsin, thrombin and papain, and endolys C.

5. Method according to Claim 1, **characterized in that** the protease inhibition step is carried out by addition of an antiprotease chemical compound.

6. Method according to Claim 5, **characterized in that** the antiprotease may be chosen from pepstatin, leupeptin, fetal bovine serum, phenylmethylsulfonyl fluoride.

7. Method according to one of Claims 1 to 6, **characterized in that** the first filter (F1) is provided with pores having a size of between 1 µm and 6 µm.

8. Method according to one of Claims 1 to 7, **characterized in that** the second filter (F2) is provided with pores having a size of between 0.4 µm and 0.8 µm.

9. Method according to one of Claims 1 to 8, **characterized in that** the third step is carried out using a preparation device which comprises at least:
- the housing comprising at least one opening,
- a first channel (14) made in said housing,
- a second channel (15) made in said housing,
- a chamber (13) into which the first channel and the second channel open,
- a filter (16) separating said chamber into two separate spaces so as to define a first space (130) into which said first injection channel opens and a second space (131) into which said second channel opens, said filter having a porosity suitable for retaining said biological species to be analysed.

10. Use of the method as defined in one of Claims 1 to 9, for preparing a specimen containing biological species to be analysed according to an analysis technique chosen from q-PCR, LAMP, sequencing, and immunological detection. This
